(19) Europäisches Patentamt
European Patent Office
Office européen des brevets

(11) **EP 4 597 088 A1**

(12) **EUROPEAN PATENT APPLICATION**

(43) Date of publication:
06.08.2025 Bulletin 2025/32

(21) Application number: 25152406.2

(22) Date of filing: 17.01.2025

(51) International Patent Classification (IPC):
**G01N 23/2055** (2018.01)    **G01N 23/207** (2018.01)

(52) Cooperative Patent Classification (CPC):
**G01N 23/2055; G01N 23/207**; G01N 2223/62

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2024 JP 2024012292**

(71) Applicant: **Rigaku Corporation**
**Akishima-shi**
**Tokyo 196-8666 (JP)**

(72) Inventors:
• **TAHARA, Daisuke**
**Tokyo, 196-8666 (JP)**
• **ITO, Kazuki**
**Tokyo, 196-8666 (JP)**
• **OMOTE, Kazuhiko**
**Tokyo, 196-8666 (JP)**

(74) Representative: **Lambacher, Michael et al**
**V. Füner Ebbinghaus Finck Hano**
**Patentanwälte**
**Mariahilfplatz 3**
**81541 München (DE)**

(54) **CALCULATION APPARATUS, SYSTEM, METHOD, AND PROGRAM**

(57)    A calculation apparatus, a system, a method, and a program for simply estimating an orientation direction from a two-dimensional diffraction image and calculating a structural model of a polymer are provided. A calculation apparatus 400 for calculating a structural model of a polymer comprises a data acquiring section 410 for acquiring a two-dimensional diffraction image and crystal structure data, an initial structural model generating section 420 for generating an initial structural model including one crystallite, and a crystallite direction calculating section 430 for calculating a direction of the crystallite, wherein the direction of the crystallite is calculated based on a two-dimensional diffraction image calculated from the initial structural model and the measured two-dimensional diffraction image.

FIG. 3

EP 4 597 088 A1

**Description**

RELATED ART

Field of the Invention

The present invention relates to a calculation apparatus, a system, a method, and a program for calculating a structural model of a polymer.

Description of the Related Art

The properties of polymer materials are closely related to the orientation distribution. WAXD (Wide Angle X-ray Diffraction) method is widely used as a method for measuring the orientation distribution of a polymer material. However, since the crystallization of the polymer material is lower than that of the low-molecular material, there is a problem that the diffraction points are few in the diffraction image and diffuse diffraction from the non-crystal is intense, and therefore, the same analysis as that of the low-molecular material is difficult. Therefore, there is a demand for a unique method for analyzing polymer material.
Non-Patent Document 1 describes methods for estimating electron-density profiles in real space from diffracted images by the RMC (Reverse Monte Carlo) method.
Non-Patent Document 2 describes a method for applicating the method of Non-Patent Document 1 to the polymer material to obtain a three-dimensional macromolecular structure that reproduces an actual WAXD image by simulating with the RMC method.
Non-Patent Document 3 describes a method for obtaining an orientation direction and an orientation distribution by generating a structural model of a uniaxially oriented sample and comparing a diffraction image calculated from the structural model with a measured diffraction image.

Non-Patent Document

Non-patent Document 1: R. L. McGreedy, L. Pusztai, "Reverse Monte Carlo Simulation: A New Technique for the Determination of Disordered Structures", Molecular Simulation, 1988, 1, 6, p. 359-367
Non-patent Document 2: Daisuke Tawara, Kohji Tashiro, "Development of Polymer Three-Dimensional Higher Structural Analysis Based on Computer Simulation Technique of Measured Wide-Angle and Small-Angle X-Ray Scattering Data", Proceedings of the Society of Polymer Science Japan, 2018, 67, 1, 1Pe027
Non-patent Document 3: Y.Zhang, et. al., "Uncovering three-dimensional gradients in fibrillar orientation in an impact-resistant biological armour", Scientific Reports 6, 26249 (2016)

SUMMARY OF THE INVENTION

In the analysis in Non-Patent Document 2, the problem is that the time required to reach the equilibrium state becomes longer due to the large number of degrees of freedom and the large amount of calculation relating to the change of the individual degrees of freedom.
In Non-Patent Document 3, the analysis target is limited to a uniaxially oriented sample such as a fiber. That is, only a two-dimensional orientation distribution is assumed. However, in order to analyze the structural of an industrially important macromolecular film, it is necessary to clarify the three-dimensional orientation distribution. Further, since the analysis is performed assuming the fiber orientation by uniaxial stretching, it is not possible to express a complicated orientation distribution unlike Non-Patent Document 2 using simulation by the RMC method.
As a result of intensive studies, the present inventors have found methods for easily estimating the direction orientation from a WAXD image even in a sample having a three-dimensional orientation distribution. In addition, they have found that the amount of calculation can be reduced by simulating with the RMC method using the estimated orientation direction as an initial value and the total optimum solution can be obtained, and they have completed the present invention.
The present invention has been made in view of such circumstances, and an object of the present invention is to provide a calculation apparatus, a system, a method, and a program for easily estimating an orientation direction from a two-dimensional diffraction image and calculating a structural model of a polymer.

(1) In order to achieve the above object, a calculation apparatus of the present invention is a calculation apparatus for calculating a structural model of a polymer, and includes a data acquiring section for acquiring a two-dimensional diffraction image and crystal structure data, an initial structural model generating section for generating an initial structural model including one crystallite, and a crystallite direction calculating section for calculating a direction of the crystallite, wherein the direction of the crystallite is calculated based on a two-dimensional diffraction image calculated from the initial structural model and the measured two-dimensional diffraction image.

(2) Further, in the calculation apparatus of the present invention, the crystallite direction calculating section calculates a degree of coincidence between two-dimensional diffraction images calculated from the plurality of initial structural models corresponding to a plurality of directions of the one crystallite and the measured two-dimensional diffraction image and calculates the direction of the crystallite based on the calculated degree of coincidence.

(3) Further, the calculation apparatus of the present invention further comprises a pre-calculation structural model generating section for generating a pre-calculation structural model in which the directions of the plurality of crystallites are the same direction for the structural model having the plurality of crystallites, and a calculation executing section for changing the structural model based on the degree of coincidence between the two-dimensional diffraction image calculated from the structural model and the measured two-dimensional diffraction image, wherein the calculation executing section calculates a post-calculation structural model in which the degree of coincidence satisfies a convergence condition using the pre-calculation structural model as a first structural model.

(4) Further, the calculation apparatus of the present invention further comprises a crystal component extracting section for extracting a crystal component of the measured two-dimensional diffraction image, wherein the crystallite direction calculating section calculates the direction of the crystallite using the crystal component of the measured two-dimensional diffraction image.

(5) Further, in the calculation apparatus of the present invention, the direction of the crystallite is a three-dimensional vector.

(6) Further, the calculation executing section calculates the post-calculation structural model using the RMC method.

(7) Further, in the calculation apparatus of the present invention, the pre-calculation structural model generating section selectively generates either the pre-calculation structural model or a second pre-calculation structural model in which directions of the plurality of crystallites are randomly arranged with respect to the structural model, and the calculation executing section calculates the post-calculation structural model using either the pre-calculation structural model or the second pre-calculation structural model as a first structural model.

(8) Further, the system of the present invention comprises an X-ray diffraction apparatus comprising an X-ray generator for generating X-rays, a detector for detecting X-rays, and a sample stage for controlling the rotation of the sample, and the calculation apparatus according to any one of (1) to (7) described above.

(9) Further, the method of the present invention is a method for calculating a structural model of a polymer and comprises the following steps of acquiring a two-dimensional diffraction image and crystal structure data, generating an initial structural model including one crystallite, and calculating a direction of the crystallite, wherein in the step of calculating the direction of the crystallite, the direction of the crystallite is calculated based on a two-dimensional diffraction image calculated from the initial structural model and the measured two-dimensional diffraction image.

(10) Further, the program of the present invention is a program for calculating a structural model of a polymer and causes a computer to execute the following processing acquiring a two-dimensional diffraction image and crystal structure data, generating an initial structural model including one crystallite, and calculating a direction of the crystallite, wherein in the calculating a direction of the crystallite, the direction of the crystallite is calculated based on a two-dimensional diffraction image calculated from the initial structural model and the measured two-dimensional diffraction image.

Brief Description of the Drawings

FIG.1 is a schematic diagram showing an example of the configuration of the calculation system for the present invention.

FIG.2 is a block diagram showing an example of the configuration of the control apparatus.

FIG.3 is a block diagram showing an example of the configuration of the calculation apparatus.

FIG.4 is a schematic diagram showing a state in which a polymer, which is replaced with a cylindrical electron density, is placed in a unit cell.

FIG.5 is a schematic diagram showing an example of a direction of a crystallite.

FIG.6 is a block diagram showing a modified example of the configuration of the calculation apparatus.

FIG.7 is a block diagram showing a modified example of the configuration of the calculation apparatus.

FIGS. 8A to 8D are schematic diagrams of two-dimensional diffraction images showing respective steps of an example extracting the crystalline components of the measured two-dimensional diffraction image.

FIG.9 is a flowchart showing an example of the operation of the calculation apparatus.

FIG.10 is a flowchart showing a modified example of the operation of the calculation apparatus.

FIG.11 is a flowchart showing a modified example of the operation of the calculation apparatus.

FIG. 12A to 12C are schematic diagrams showing the measured two-dimensional diffraction image of the HDPE sample, the two-dimensional diffraction image of the post-calculation structural model in the practical example and the two-dimensional diffraction image in the comparative example, respectively.

FIG.13 is a graph showing changes in Fitness of a practical example and a comparative example.

FIGS. 14A and 14B are a projection diagram showing the distribution of orientation directions in the initial-state and a projection diagram showing the distribution of orientation directions when the convergence condition is satisfied, respectively, in the practical example.

FIGS. 15A and 15B are a projection diagram showing the distribution of orientation directions in the initial-state and a projection diagram showing the distribution of orientation directions when the convergence condition is satisfied, respectively, in the comparative example.

## DETAILED DESCRIPTION OF THE INVENTION

Next, embodiments of the present invention are described with reference to the drawings. To facilitate understanding of the description, the same reference numerals are assigned to the same components in the respective drawings, and duplicate descriptions are omitted.

[Principle]

The RMC method can obtain the approximate solution more efficiently than the full search by appropriately sampling in the multi-degree-of-freedom system. However, when the calculation by the RMC method is applied to the estimation of the crystal orientation distribution of the polymer material, due to the large number of degrees of freedom and the large amount of calculation concerning the change of the individual degrees of freedom, the time required to reach equilibrium by the Metropolis method becomes long. Therefore, it is not possible to meet the demand in a case where it is required to obtain an analysis result of a large number of samples at a high speed.

The present inventors have noticed that many samples used in the development site of polymer materials have a relatively high degree of orientation. They found that it is possible to shorten the time until the final result is obtained by starting the execution with an initial value being not a random orientation distribution and being estimated from the two-dimensional diffraction image acquired by measuring orientation direction which is highly likely to be oriented as many as possible crystallites of the sample, when the orientation distribution is estimated by the RMC method for such a sample.

That is, the method of the present invention is a method for performing the RMC method as an initial value using the crystallite direction in which the potential is minimized among the structural models assuming all the crystallites in the same direction is detected. The orientation distribution obtained from such an initial value is highly likely to avoid a local minimum for a sample having a relatively high degree of orientation, and it is highly likely that the crystal orientation distribution of the sample is estimated with high accuracy. Further, the method for estimating the initial value is considered to be a method suitable for estimating the initial value prior to applying the RMC method, since the method is similar to the RMC method of the structural model in which all crystallites are restricted in the same direction. The method of calculating the direction of the crystallite of the initial value and the method of calculating the orientation distribution according to the present invention are described in detail in the embodiments.

[Embodiment]

[Whole System]

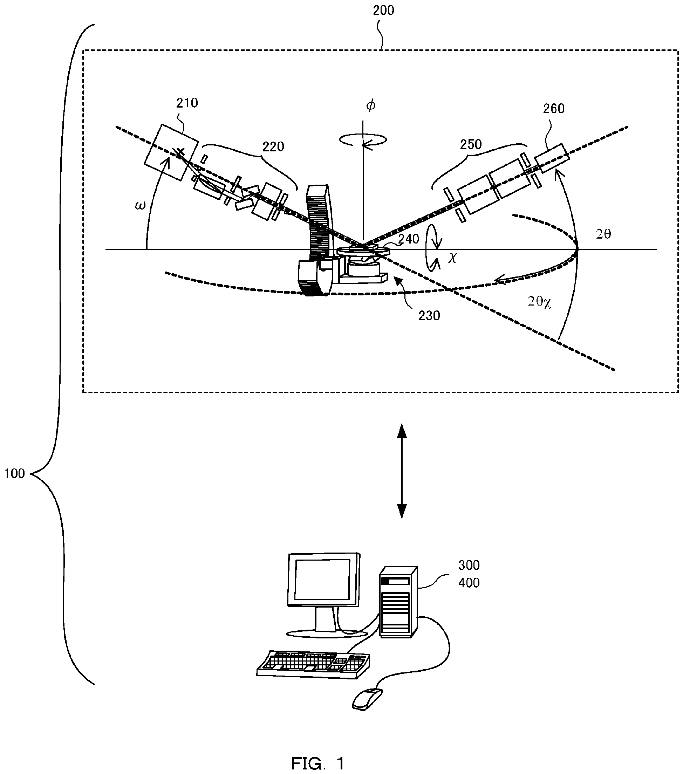

FIG. 1 is a schematic diagram showing an example of the configuration of a calculation system 100 of the present invention. The calculation system 100 comprises an X-ray diffraction apparatus 200, a control apparatus 300, and a calculation apparatus 400. The X-ray diffraction apparatus 200 makes X-rays incident on a sample and constitutes an optical system for detecting diffracted X-rays generated from the sample, and the optical system comprises a goniometer. Incidentally, the configuration shown in FIG. 1 is one example, and thus a variety of other configurations may be adopted.

In FIG. 1, the control apparatus 300 and the calculation apparatus 400 are described as the same PC. However, the method of the present invention, as is described below, regardless of the X-ray diffraction apparatus 200 and the control apparatus 300, by acquiring a two-dimensional diffraction image, it is possible to calculate the direction of the crystallite or calculate the calculation structural model, the calculation apparatus 400 may be configured as a different apparatus from the control apparatus 300. Hereinafter, a case where the control apparatus 300 and the calculation apparatus 400 are configured as different apparatuses is described.

[X-ray Diffraction Apparatus]

The X-ray diffraction apparatus 200 comprises at least an X-ray generating section 210 for generating X-rays from an X-ray focal point or X-ray source, a sample stage 240 on which a sample is installed and controls rotation of the sample, and a detector 260 for detecting X-rays. The X-ray diffraction apparatus 200 may comprise an incident-side optical unit 220, a goniometer 230 or an emitting side optical unit 250. Since the X-ray generating section 210, the incident-side optical unit 220, the goniometer 230, the sample stage 240, the emission-side optical unit 250 and the detector 260 constituting the X-ray diffraction apparatus 200 need only be general, a detailed description thereof is omitted.

For the wavelength of the X-ray source of the X-ray generating section 210, such as CuK$\alpha$ ray and MoK$\alpha$ ray used in general diffraction measurement may be used. X-rays generated from the X-ray source are irradiated onto a sample by a dotted irradiation field, and the generated diffraction X-rays are detected by the detector 260 (two-dimensional detector). Methods to make a dotted irradiation field include slits, collimators, mirrors, polycapillaries and the like.

[Control Apparatus]

The control apparatus 300 is connected to the X-ray diffraction apparatus 200 to control the X-ray diffraction apparatus 200 and process, store, and display the acquired data. Within the calculation system 100, if the calculation apparatus 400 does not generate a pre-calculation structural model or calculate a post-calculation structural model, the control apparatus 300 or other apparatus (e.g., a structural model generating apparatus) may have the capability to generate a pre-calculation structural model or calculate a post-calculation structural model.

The control apparatus 300 is constituted from a computer formed by connecting CPU (Central Processing Unit/Central Processor), ROM (Read Only Memory), RAM (Random Access Memory) and a memory to a bus. The control apparatus 300 may be a PC terminal or a server on the cloud. Not only the whole apparatus but also part of the apparatus or some functions of the apparatus may be provided on the cloud.

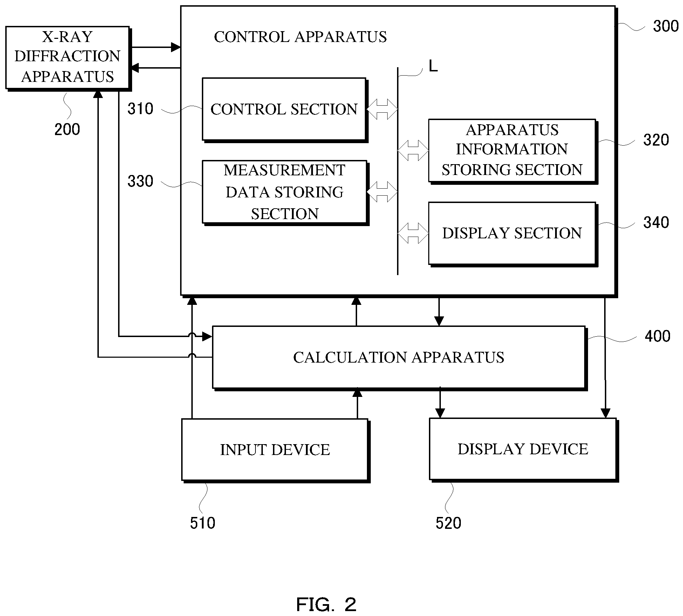

FIG. 2 is a block diagram showing an example of the configuration of the control apparatus 300. The control apparatus 300 comprises a control section 310, an apparatus information storing section 320, a measurement data storing section 330 and a display section 340. Each section can transmit and receive information via the control bus L. The input device 510 and the display device 520 are connected to CPU of the control apparatus 300 via an appropriate interface. The input device 510 is, for example, a keyboard or a mouse and performs input to the control apparatus 300. The display device 520 is, for example, a display, and displays measurement data, a measured two-dimensional diffraction image, crystal structure data of a sample, a two-dimensional diffraction image calculated from an initial structural model and the like.

The control section 310 controls the operations of the X-ray diffraction apparatus 200. The apparatus information storing section 320 stores apparatus information acquired from the X-ray diffraction apparatus 200. The apparatus information may include information about the X-ray diffraction apparatus 200, such as apparatus name, source type, wavelength, background, etc.

The measurement data storing section 330 stores measurement data including a two-dimensional diffraction image acquired from the X-ray diffraction apparatus 200. In addition to the measurement data, necessary information such as information on the X-ray diffraction apparatus 200 such as the source type, the wavelength, and the background, crystal structure data of the sample, shape, arrangement, type of constituent elements, composition, and absorption coefficient may be stored. The display section 340 causes the display device 520 to display the measurement data, the measured two-dimensional diffraction image, the crystal structure data of the sample, the two-dimensional diffraction image calculated from the initial structural model and the like. Thus, the measurement data, etc. can be confirmed by a user. In addition, the user can instruct and designate the control apparatus 300, the calculation apparatus 400 and the like based on the measurement data and the like.

[Calculation Apparatus]

The calculation apparatus 400 calculates a structural model of the polymer. The calculation apparatus 400 is configured from a computer formed by connecting CPU, ROM, RAM and a memory to a bus. The calculation apparatus 400 may be a PC terminal or a server on a cloud. Not only the whole apparatus but also part of the apparatus or some functions of the apparatus may be provided on the cloud. The calculation apparatus 400 may be connected to the X-ray diffraction apparatus 200 via the control apparatus 300, for example.

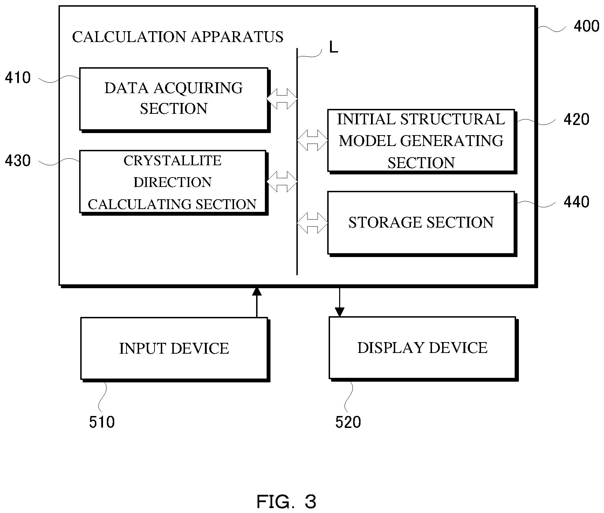

FIG. 3 is a block diagram showing an example of the configuration of the calculation apparatus 400. The calculation apparatus 400 includes a data acquiring section 410, an initial structural model generating section 420, a crystallite direction calculating section 430, and a storage section 440. Each section can transmit and receive information via the control bus L.

The input device 510 and the display device 520 are connected to CPU of the calculation apparatus 400 via an appropriate interface. In this case, the input device 510 and the display device 520 each may differ from one connected to the control apparatus 300. The input device 510 is, for example, a keyboard or a mouse and performs input to the calculation apparatus 400. The display device 520 is, for example, a display, and displays measurement data, a measured two-dimensional diffraction image, a two-dimensional diffraction image calculated from an initial structural model or a structural model, crystal structure data, an initial structural model, a crystallite direction, a pre-calculation structural model, a post-calculation structural model, and the like.

The data acquiring section 410 acquires two-dimensional diffraction images and crystal structure data. The crystal structure data includes the type and atomic coordinates of atoms in the crystallite, the direction of the crystallite, and crystal structure parameters (unit cell, temperature factor, and the like).

The two-dimensional diffraction images acquired by the data acquiring section 410 from the X-ray diffraction apparatus 200, the control apparatus 300 or another apparatus may be only the measured two-dimensional diffraction images. When the two-dimensional diffraction images acquired by the data acquiring section 410 from the control apparatus 300 or the X-ray diffraction apparatus 200 is only the measured two-dimensional diffraction images, the initial structural model generating section 420 calculates the two-dimensional diffraction image from the generated initial structural model. In this case, the data acquiring section 410 acquires the two-dimensional diffraction image calculated from the initial structural model (also referred to as a two-dimensional diffraction image corresponding to the initial structural model) from the initial structural model generating section 420.

The two-dimensional diffraction image acquired by the data acquiring section 410 may include a measured two-dimensional diffraction image and a two-dimensional diffraction image corresponding to the initial structural model. For example, in a case where the crystal structure data and the two-dimensional diffraction image corresponding to the initial structural model are stored in a database or the like, the two-dimensional diffraction image corresponding to the initial structural model may be acquired.

The initial structural model generating section 420 generates an initial structural model including one crystallite. When the three-dimensional orientation is fixed, the initial structural model is a structural model including one crystallite but may be a structural model in which the directions of a plurality of crystallites are the same direction. However, the initial structural model is used to calculate a two-dimensional diffraction image corresponding to the direction of the one crystallite for comparison with the measured two-dimensional diffraction image. However, the diffraction intensity of the calculated two-dimensional diffraction image is only increased even in a structural model in which the directions of a plurality of crystallites are the same direction. Therefore, it is simple and preferable for the initial structural model to be a structural model that includes only one crystallite, which is the smallest unit of the polymer material.

The crystallite direction calculating section 430 calculates the direction of the crystallite. The crystallite direction calculating section 430 may calculate the crystallite size in addition to the crystallite direction. The direction of the crystallite or the size of the crystallite is calculated based on the two-dimensional diffraction image calculated from the initial structural model and the measured two-dimensional diffraction image. By calculating the size of the crystallite based on the two-dimensional diffraction image calculated from the initial structural model and the measured two-dimensional diffraction image, it is possible to estimate the initial value of the size of the crystallite.

The diffraction intensity of the two-dimensional diffraction image calculated from the initial structural model is calculated from the direction of the crystallite and the crystal structure data. In the method, for example, the diffraction intensity in the reciprocal space is calculated from the crystal structure. Next, the intensity distribution in the reciprocal space is rotated in correspondence with the direction of the crystallite, and the intensity on the Ewald sphere is calculated. In this way, the diffraction intensity of the two-dimensional diffraction image corresponding to the initial structural model can be calculated. For the crystal structure data, a known crystal structure may be used. The crystallite size may be obtained from the measured diffraction width.

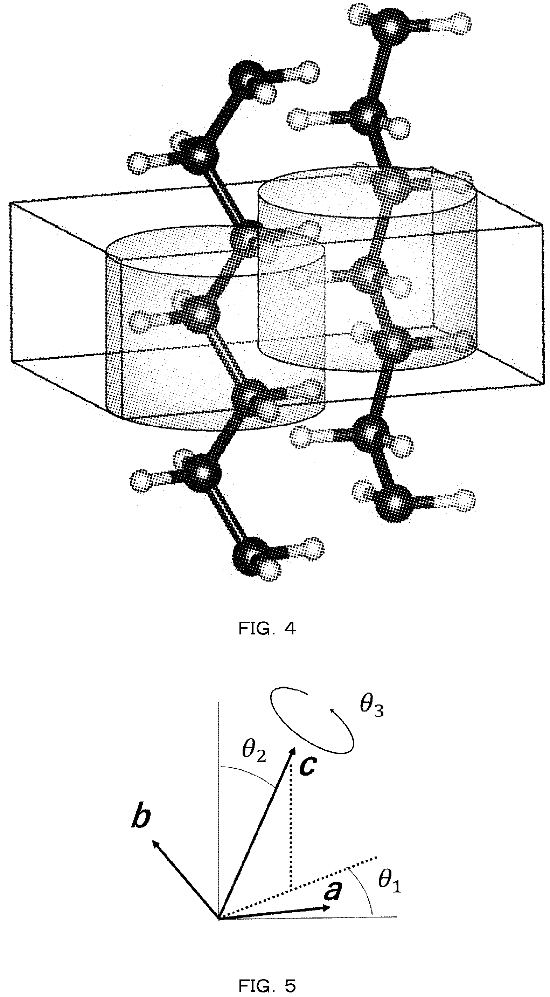

In order to calculate the diffraction intensity of a two-dimensional diffraction image from an initial structural model or a structural model, it is necessary to know the atomic coordinates of a specific crystal. In practice, however, the crystal structure may not be known. In this case, it is necessary to determine the atomic coordinates, but it is difficult to determine the optimum coordinates by moving the individual atomic coordinates because of the number of diffraction points of the two-dimensional diffraction image of the polymers. For this reason, as a preliminary step of determining a precise crystal structure, a method of replacing a polymer with, for example, a cylindrical electron density to placing the polymer density in a unit cell is conceivable. FIG.4 is a schematic diagram showing a state in which a polymer, which is replaced with a cylindrical electron density, is placed in a unit cell. In the example of FIG. 4, instead of the atomic coordinates of the polymer, the diffraction intensity of the two-dimensional diffraction image is calculated using a cylindrical electron density distribution placed in the unit cell as an initial structural model or a structural model. Incidentally, the shape of the electron density to replace is not limited to a cylindrical shape, it may be formed by a method that can be coarse graining. For example, it is possible to form various shapes such as polygonal columnar, cylindrical, polygonal tubular, plate-like, linear, spherical, elliptical spherical, etc. These shapes may also be used in combination.

By using such a method, a two-dimensional diffraction image can be calculated by the approximated electron density distribution. In addition to the orientation of the crystallites, the RMC analysis using the lattice constant of the unit cell as a parameter can be performed to roughly estimate the lattice constant. This is the first step, and it is also possible to refine the structure while increasing the parameters, for example, to reproduce the fiber period by providing a periodic density distribution along the cylinder.

The direction of the crystallite is preferably a three-dimensional vector. The three-dimensional vector indicating the direction of the crystallite includes a method of displaying an Euler angle. FIG. 5 is a schematic diagram showing an example of a direction of a crystallite. FIG. 5 represents the direction of the one crystallite with an Euler angle $(\theta_1, \theta_2, \theta_3)$ in the coordinate fixed to the sample. Note that $0° \leqq \theta_1 < 360°$, $0° \leqq \theta_2 < 180°$, $0° \leqq \theta_3 < 360°$. The three-dimensional vector indicating the direction of the crystallite may be represented by a display method other than the Euler angle. When the direction of the crystallite is represented with a three-dimensional vector, the orientation distribution of the structural model described later is represented as a set of three-dimensional vectors corresponding to the number of crystallites. It is not necessary to consider the correlation depending on the positions of the crystallites. If one-axis symmetry of the sample is assumed, the rotation average about the axis may be calculated and the orientation may be represented by a two-dimensional vector.

It is preferable that the direction of the crystallite calculating section 430 calculates the degree of coincidence between the two-dimensional diffraction image calculated from the plurality of initial structural models corresponding to the plurality of directions of the one crystallite and the measured two-dimensional diffraction image and calculates the direction of the crystallite based on the calculated degree of coincidence. For the direction of the crystallite to be calculated may be selected the direction of the best degree of coincidence, or may be selected two directions, the best direction and the second best direction of the degree of coincidence, among the respective direction degrees of coincidence. When selecting two directions of the crystallites to be calculated, the orientation distribution of the crystallites of the pre-calculation structural model to be described later, for example, the best direction and the second best direction may be arranged in half, but it is preferable to arrange in a ratio based on the intensity ratio or the value of the degree of coincidence of each two-dimensional diffraction image.

If the sample has uniaxial orientation, there may be little difference in the degree of coincidence for a plurality of crystallites in which the directions of one axis are the same and the directions of the other axes are different, and it may not be possible to determine one or two directions of the best degree of coincidence. In such a case, the direction of the same axis may be determined as the direction of the crystallite, and the direction of the remaining axes may be random. That is, the direction of the crystallite calculated based on the degree of coincidence includes that in a case where a single axis or two axes are fixed, and the remaining axis is random. Note that the above-described axis is not limited to the actual axis of the crystallite. Further, in a case where the direction of the crystallite is represented by a three-dimensional vector, the direction of the crystallite calculated based on the degree of coincidence includes that in a case where one or two values of the three-dimensional vector are limited and the remaining value is random, but also that in a case where the ratio of the two values is limited and the remaining value is random. When the direction of the crystallite is represented by an Euler angle, the direction of the crystallite calculated based on the degree of coincidence includes that in a case where the value of the specific Euler angle is limited and the value of the remaining Euler angle is random.

The plurality of directions of one crystallite refer to different directions of the crystallite. For example, when the direction of a crystallite is represented by an Euler angle $(\theta_1, \theta_2, \theta_3)$, the directions of the one crystallite are represented by different combinations of Euler angles $(\theta_1, \theta_2, \theta_3)$. The plurality of directions of one crystallite preferably includes all possible combinations depending on the resolution of the X-ray diffraction apparatus 200. For example, if the resolution of the X-ray diffraction apparatus 200 is 0.5°, they can be $\theta_1 = 0.51$ (l=0, 1, ..., 719), $\theta_2 = 0.5m$ (m=0, 1, ..., 359), $\theta_3 = 0.5n$ (n=0, 1, ..., 719) .

Various methods can be used to calculate the degree of coincidence between a plurality of two-dimensional diffraction images calculated from a plurality of initial structural models corresponding to a plurality of directions of the one crystallite and a measured two-dimensional diffraction image and calculate the direction of the crystallite based on the calculated degree of coincidence. For example, when the sum of squares of the intensity difference between the two-dimensional diffraction image calculated from a certain initial structural model and the respective spots of the measured two-dimensional diffraction image is the degree of coincidence (degree of divergence) of the initial structural model, the direction of the crystallite corresponding to the initial structural model whose value is smaller can be calculated as an initial value. In general, the degree of coincidence is a reference for determining that a larger value is better, and the degree of divergence is a reference for determining that a smaller value is better. However, the term "degree of coincidence" in the present specification is also intended to include the degree of divergence.

The degree of coincidence (Fitness) between the two-dimensional diffraction image calculated from the initial structural model and the measured two-dimensional diffraction image can be calculated, for example, using the following formula (1). It is to be noted that $q_x, q_y$: the position on WAXD images, $I_{obsd}$: measured intensity, $I_{calcd}$: calculated intensity, and a: scaling factor obtained by the least-square method. The formula (1) is a reference for determining that a smaller Fitness is better. Note that the mathematical expression for calculating the degree of coincidence between the two-dimensional diffraction image calculated from the initial structural model and the measured two-dimensional diffraction image is not limited to the formula (1).

$$Fitness = \sum_{q_x,q_y} \left( I_{obsd}(q_x, q_y) - a I_{calcd}(q_x, q_y) \right)^2 \Big/ \sum_{q_x,q_y} I^2_{obsd}(q_x, q_y) \qquad \cdots (1)$$

The storage section 440 stores required information among the data acquired by the data acquiring section 410, the initial structural model generated by the initial structural model generating section 420, the diffraction intensity of the two-dimensional diffraction image corresponding to the initial structural model, the plurality of values of the degrees of coincidence and the direction of the crystallites calculated by the crystallite direction calculating section 430, the pre-calculation structural model generated by the pre-calculation structural model generating section 450 described later, the structural model changed and the post-calculation structural model generated by the calculation executing section 460 and the like. Further, the storage section 440 stores required information among mathematical expressions for calculating the diffraction intensity of the two-dimensional diffraction image from the initial structural model or the structural model, mathematical expressions for calculating the degree of coincidence between the two-dimensional diffraction image calculated from the initial structural model or the structural model and the measured two-dimensional diffraction image, numerical values for calculating these, conditions and the like.

In order to clarify the three-dimensional orientation, it is necessary to calculate the orientation direction from a plurality of two-dimensional diffraction images. Therefore, in order to estimate the initial value of the orientation direction to be set in the initial structural model, specialized knowledge on polymer materials and diffraction methods is usually required. However, by using the method of the present invention, the initial value can be estimated automatically, so that no specialized knowledge is required.

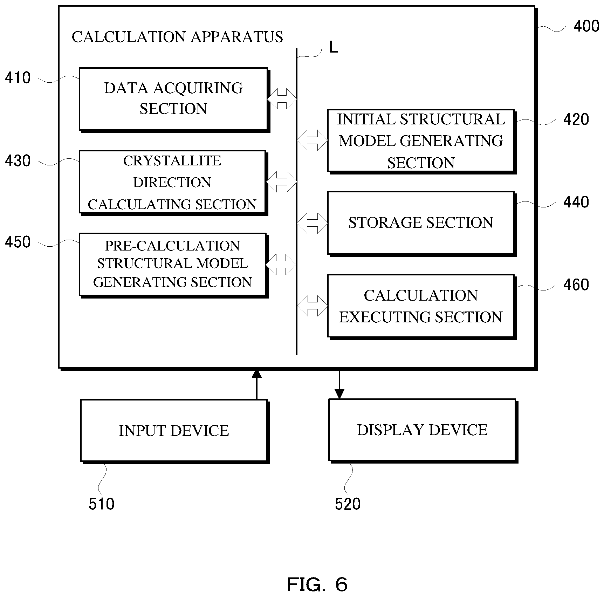

FIG.6 is a block diagram showing a modified example of the configuration of the calculation apparatus 400. As shown in FIG. 6, the calculation apparatus 400 preferably includes a pre-calculation structural model generating section 450 and a calculation executing section 460.

The pre-calculation structural model generating section 450 generates a pre-calculation structural model in which directions of a plurality of crystallites are the same direction with respect to a structural model including a plurality of crystallites. The structural model in the present specification means a model having a plurality of crystallites. Further, in the pre-calculation structural model, the directions of the plurality of crystallites being the same means that the directions of the crystallites calculated by the crystallite direction calculating section 430 are the same. That is, the directions of the plurality of crystallites of the pre-calculation structural model include that in a case where a single axis or two axes are fixed, and the remaining axis is random. The pre-calculation structural model generating section 450 sets the number of crystallites in the RMC method. The number of crystallites has a trade-off relationship between the calculation accuracy and the calculation time of the orientation distribution that can be calculated from the calculated structural model, and therefore, it is preferable to set an appropriate number of crystallites according to the target calculation accuracy. The pre-calculation structural model generating section 450 may set the number of crystallites based on a value such as a calculation time specified by the user.

When the crystallite direction calculating section 430 calculates the directions of the two crystallites as the initial values, the pre-calculation structural model generated by the pre-calculation structural model generating section 450 may be a structural model in which the directions of the plurality of crystallites are any of the directions of the two crystallites. Such a pre-calculation structural model is also included in the pre-calculation structural model in which the directions of the plurality of crystallites are the same direction. In this case too, the direction of one or two crystallites may include that in a case where one or two axes are fixed and the remaining axis is random, etc.

The calculation executing section 460 changes the structural model based on the degree of coincidence between the two-dimensional diffraction image calculated from the structural model and the measured two-dimensional diffraction image. Further, the calculation executing section 460 calculates a post-calculation structural model in which the degree of coincidence satisfies the convergence condition using the pre-calculation structural model as an initial value (a first structural model). Thus, a post-calculation structural model that incorporates the orientation of crystallites that are likely to be included in the sample can be obtained, and a measured two-dimensional diffraction image becomes interpretable by such a post-calculation structural model.

For the convergence condition, various conditions can be set. For example, for a mathematical formula for determining a convergence condition, a mathematical expression for determining a degree of coincidence may directly be used, or another mathematical expression using a series of values of degrees of coincidence obtained from the mathematical expression may be used. For example, when the absolute value of the degree of coincidence becomes equal to or less than the threshold value, it may be determined that the convergence condition is satisfied, and the process may be terminated. Further, for example, the standard deviation of the degree of coincidence in the latest predetermined number of times is acquired, when the standard deviation becomes equal to or smaller than a predetermined threshold value, it may be determined that the convergence condition is satisfied, and the process may be terminated. Further, for example, (a) the amount of change in the average value of the degree of coincidence and (b) the standard deviation of the degree of coincidence in the latest predetermined number of times are acquired, and when the ratio of (a) and (b) becomes equal to or less than a predetermined threshold value, it may be determined that the convergence condition is satisfied, and the process may be terminated.

The method of Geweke is shown as an example of convergence determination by comparing the change amount of the mean value with the standard deviation. The method of Geweke is a method that the convergence may be determined when the absolute value of Z is sufficiently smaller than 1 in the following formula (2). Where $G_1$: mean of the first 10% of the sample, $G_2$: mean of the last 50% of the sample, $V_1$: variance of the first 10% of the sample, and $V_2$: variance of the last 50% of the sample. In this case, the sample may be, for example, a sequence of values of a series of degrees of coincidence obtained from the mathematical expression for obtaining a degree of coincidence.

$$Z = \frac{G_1 - G_2}{\sqrt{V_1 + V_2}} \qquad \cdots \ (2)$$

The calculation executing section 460 preferably calculates the post-calculation structural model using the RMC method. The RMC method is a method for estimating a structural model which reproduces the measured value by changing the atomic position of a given structural model and the direction of crystallites using random numbers. The RMC method is useful for solving complex optimization problems because it has a wide search space, and a global minimum solution is obtained. When the RMC method is applied to the present embodiment, the post-calculation structural model is calculated using the pre-calculation structural model in which the directions of crystallites coincide with each other as an initial value. For the optimization method, an evolutionary algorithm may be used.

Various methods can be used to calculate the two-dimensional diffraction image from the structural model. For example, the intensity of the two-dimensional diffraction image can be calculated from the direction of one crystallite of the structural model and the crystal structure data, and the two-dimensional diffraction image can be calculated from the structural model by superimposing the two-dimensional diffraction image for all the crystallites.

The pre-calculation structural model generating section 450 preferably selectively generates either a pre-calculation structural model or a second pre-calculation structural model in which directions of a plurality of crystallites are randomly arranged with respect to the structural model. The second pre-calculation structural model is a structural model in which all values of the directions of the plurality of crystallites are according to a random arrangement. At this time, the calculation executing section 460 calculates the post-calculation structural model using either the pre-calculation structural model or the second pre-calculation structural model as an initial value.

Thus, it is possible to calculate a post-calculation structural model using a pre-calculation structural model in which the directions of a plurality of crystallites are the same direction as an initial value in a sample in which the degree of orientation is considered to be high. In addition, in a sample in which the degree of orientation is considered to be low, a post-calculation structural model can be calculated using a second pre-calculation structural model in which the directions of a plurality of crystallites are randomly arranged as an initial value. As a result, it is possible to reduce the time for calculating the post-calculation structural model even for a sample that is considered to have a high orientation degree and a sample that is considered to have a low orientation degree.

The selection of the pre-calculation structural model or the second pre-calculation structural model may be performed according to an instruction of the user. In addition, the selection of the pre-calculation structural model or the second pre-calculation structural model may be determined by the calculation apparatus 400 based on a numerical value or the like of the degree of coincidence between the two-dimensional diffraction image calculated from the initial structural model and the measured two-dimensional diffraction image. For example, a degree of coincidence between a two-dimensional diffraction image calculated from a plurality of initial structural models corresponding to a plurality of directions of one crystallite and a measured two-dimensional diffraction image may be calculated, and in a case where there is no direction of a crystallite in which the degree of coincidence is determined to be sufficiently high, the second pre-calculation structural model may be selected.

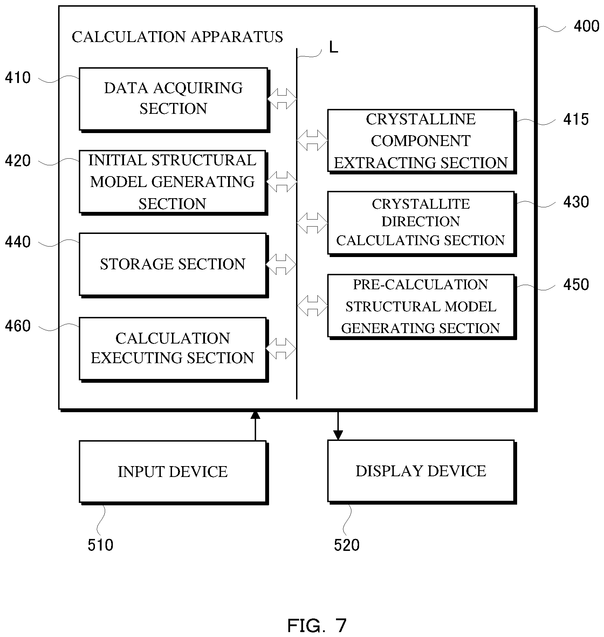

FIG.7 is a block diagram showing a modified example of the configuration of the calculation apparatus 400. As shown in FIG. 7, the calculation apparatus 400 preferably includes a crystal component extracting section 415. Although in FIG. 7, the pre-calculation structural model generating section 450 and the calculation executing section 460 are further comprised, the configuration in FIG. 3 may further comprise the crystal component extracting section 415.

The crystal component extracting section 415 extracts the crystal component of the measured two-dimensional diffraction image. When the calculation apparatus 400 comprises the crystal component extracting section 415, the crystallite direction calculating section 430 calculates the direction of the crystallite using the extracted crystal component from the measured two-dimensional diffraction image. Thus, the calculated direction of the crystallite is less susceptible to the observed non-crystalline component of the two-dimensional diffraction image.

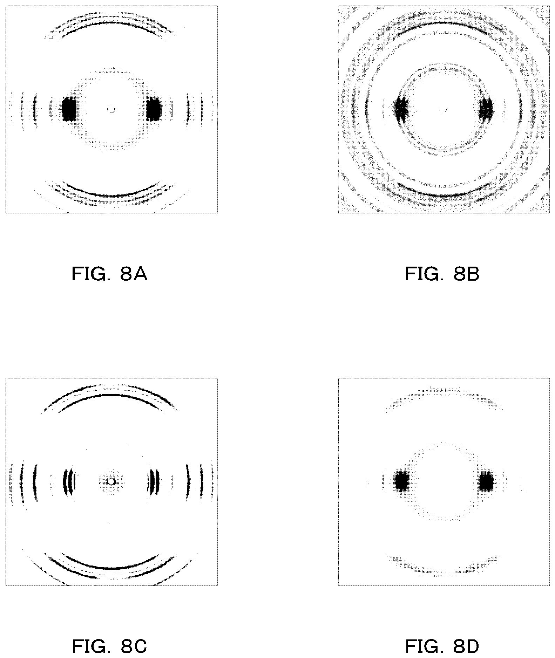

Extraction of the crystal component from the measured two-dimensional diffraction image can be performed by various methods. For example, a crystal peak is extracted from a known crystal structure and a crystal region is estimated. Next, the non-crystalline component at the position in the crystal region can be obtained by interpolation, and the crystal component can be extracted by taking a difference from the measured two-dimensional diffraction image. FIGS. 8A to 8D are schematic diagrams of two-dimensional diffraction images showing respective steps of an example extracting the crystalline components of the measured two-dimensional diffraction image. FIGS. 8A to 8D show a measured two-dimensional diffraction image, an estimated crystalline region image, an extracted crystalline component image and a remaining non-crystalline component image, respectively. FIG. 8B is a diagram showing the partial estimated to be crystalline region in a circular gray color superimposed on the measured two-dimensional diffraction image (shown in FIG. 8A) .

The calculation apparatus 400 of the present invention can calculate a two-dimensional diffraction image for a specified orientation distribution. This feature can be used to provide two-dimensional diffraction images for various orientation distributions. It is considered that a machine learning with training data using a two-dimensional diffraction image as input data and a corresponding orientation distribution as output data enables the orientation distribution to be obtained from the two-dimensional diffraction image without using time-consuming analysis such as the RMC analysis.

[Measurement Method]

A sample is placed in the X-ray diffraction apparatus 200, and X-rays are made to enter the sample under the control of the control apparatus 300, and diffracted X-rays generated from the sample are detected. If necessary, the sample stage or the goniometer is driven under a predetermined condition. Thus, the measured two-dimensional diffraction image is acquired. The X-ray diffraction apparatus 200 transmits the apparatus information and the like and the acquired two-dimensional diffraction image as measurement data to the control apparatus 300. With respect to such measurement data, the direction of the crystallite can be calculated by the calculation apparatus 400 or the calculation method of the present invention, or a post-calculation structural model for describing the measured two-dimensional diffraction image can be calculated.

[Calculation Method of Crystallite Direction]

(Description of Flow until Calculating Crystallite Direction)

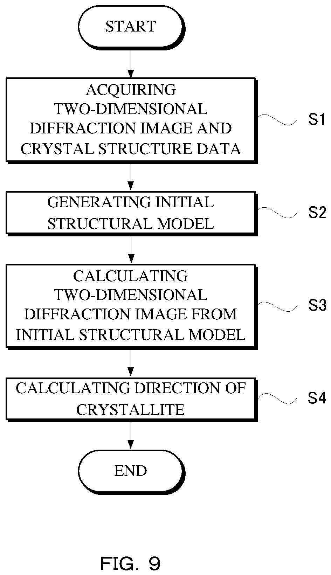

FIG. 9 is a flowchart showing an example of the operation of the calculation apparatus 400. FIG. 9 shows an example of the operation until the direction of the crystallite is calculated based on the measured two-dimensional diffraction image. First, the calculation apparatus 400 obtains a two-dimensional diffraction image and crystal structure data (step S1). Next, an initial structural model including one crystallite is generated (step S2). Next, a two-dimensional diffraction image is calculated from the initial structural model (step S3). Then, the direction of crystallite is calculated (step S4), and the process ends. The direction of the crystallite is calculated based on the two-dimensional diffraction image calculated from the initial structural model and the measured two-dimensional diffraction image. The degree of coincidence between the two-dimensional diffraction image and the measured two-dimensional diffraction image is calculated from a plurality of initial structural models corresponding to a plurality of directions of one crystallite, and the direction of the crystallite is preferable to be calculated based on the calculated degree of coincidence. If necessary, the calculated direction of the crystallite or a two-dimensional diffraction image corresponding to the direction of the crystallite may be output. In this way, it is possible to calculate the direction of the crystallite automatically in a simple manner from the measured two-dimensional diffraction image and the crystal structure data.

In this flow, the two-dimensional diffraction image is calculated from the initial structural model. However, it is not always necessary to calculate a two-dimensional diffraction image from the initial structural model, and a plurality of types of initial structural models and two-dimensional diffraction images corresponding thereto may be acquired in advance. In that case, the step of calculating the two-dimensional diffraction image from the initial structural model may not be performed. The same applies to the subsequent flowcharts.

(Description 1 of Flow until Calculating Post-Calculation Structural Model)

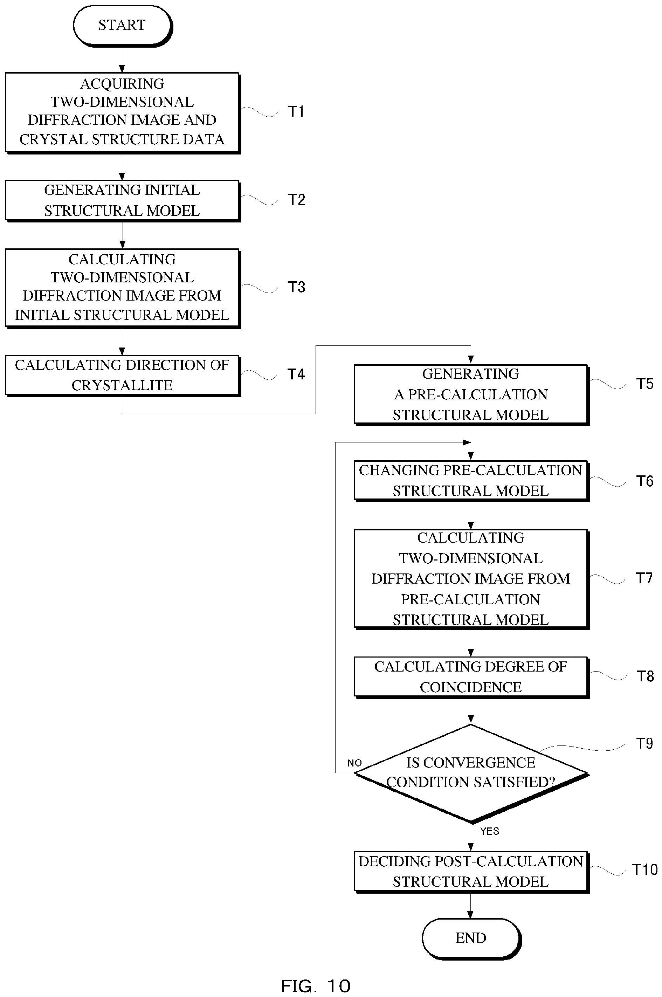

FIG.10 is a flowchart showing a modified example of the operation of the calculation apparatus 400. FIG. 10 shows an example of an operation until calculating post-calculation structural model. In the following description of the flowchart, the characteristic operation is described in detail, and the description of the operation already described may be omitted. The steps from the acquisition of the two-dimensional diffraction image and the crystal structure data (step T1) to the calculation of the direction of the crystallite (step T4) are similar to the above-described steps from the step S1 to the step S4.

Next, the calculation apparatus 400 generates a pre-calculation structural model (step T5). The pre-calculation structural model is a structural model having a plurality of crystallites, wherein all the crystallites are oriented in the calculated one or two directions of the crystallites. Next, the respective directions of the crystallites in the pre-calculation structural model are changed in a predetermined manner (step T6). Next, a two-dimensional diffraction image is calculated from the modified pre-calculation structural model (step T7). Next, the degree of coincidence between the measured two-dimensional diffraction image and the two-dimensional diffraction image calculated from the modified pre-calculation structural model is calculated (step T8). If the calculated degree of coincidence does not satisfy the convergence condition (step T9-NO), the process returns to step T6, and the process up to step T9 is performed again.

On the other hand, if the calculated degree of coincidence satisfies the convergence condition (step T9-YES), the post-calculation structural model is determined (step T10), and the process ends. If necessary, the determined post-calculation structural model or the two-dimensional diffraction image corresponding to the post-calculation structural model may be output. In this way, the post-calculation structural model can be calculated using the direction of the crystallites calculated from the measured two-dimensional diffraction image and the crystal structure data.

(Description 2 of Flow until Calculation Post-Calculation Structural Model)

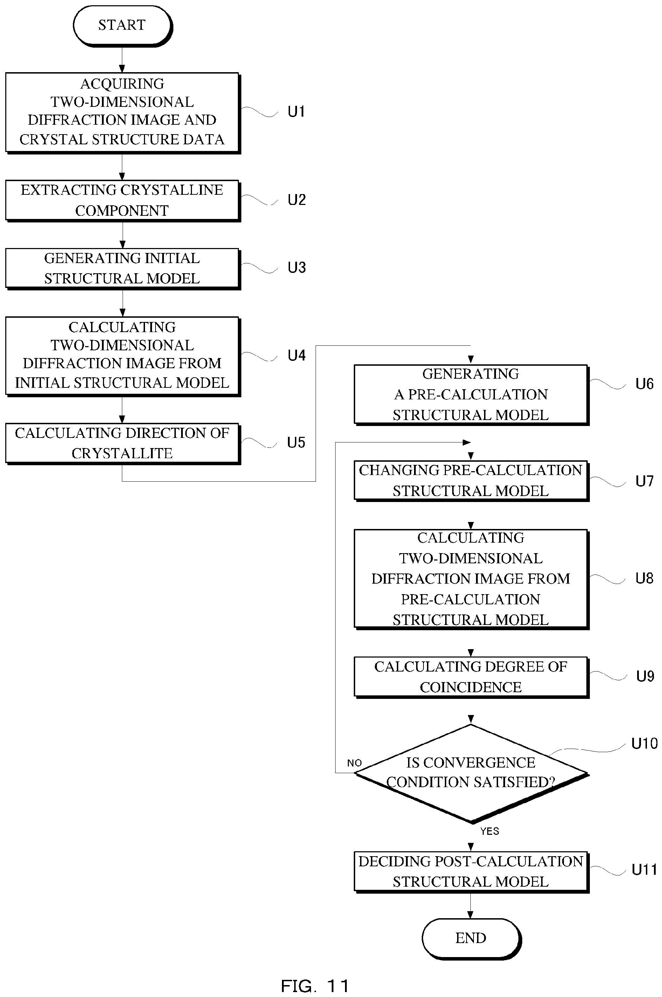

FIG.11 is a flowchart showing a modified example of the operation of the calculation apparatus 400. FIG. 11 shows a modification of the operation until calculating the post-calculation structural model. Except for the process of extracting crystalline components from the measured two-dimensional diffraction image (step U2), the operation is same as the above-described process from the step T1 to the step T10. At this time, the measured two-dimensional diffraction image used as a reference for calculating the degree of coincidence is the two-dimensional diffraction image obtained by extracting a crystal component. Thus, the direction of the crystallite is not affected by the measured non-crystalline component of the two-dimensional diffraction image.

[Example]

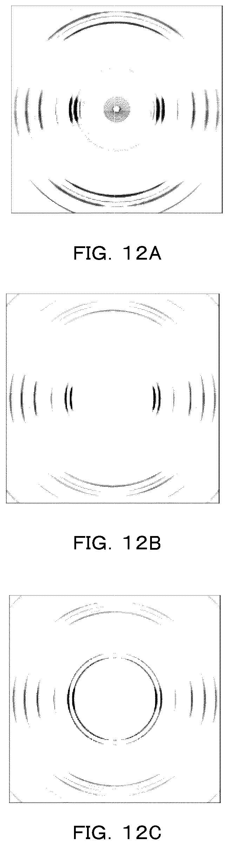

A WAXD image of a sample of high-density polyethylene (HDPE) was measured using the calculation system 100 configured as described above. Next, the image was then used to estimate the direction of the one crystallite by the method of the present invention and to determine the orientation distribution of HDPE sample using the direction as the initial value (practical example). Since HDPE sample has a uniaxial orientation, in the example, the direction in which the Euler angle $\theta_2=15°$ was estimated as the direction of the one crystallite. Therefore, the arrangement in which $\theta_2$ is limited to 15° and the remaining values are randomized is defined as the initial value. On the other hand, without using the method of the present invention, the orientation distribution of HDPE sample was determined by the common RMC method from the initial value having the random directions of the respective crystallites (comparative example). FIG. 12A to 12C are schematic diagrams showing the measured two-dimensional diffraction image of the HDPE sample, the two-dimensional diffraction image of the post-calculation structural model in the practical example and the two-dimensional diffraction image of the structural model in the comparative example, respectively. Incidentally, FIG.12A is obtained by removing the non-crystalline component from the measured WAXD image.

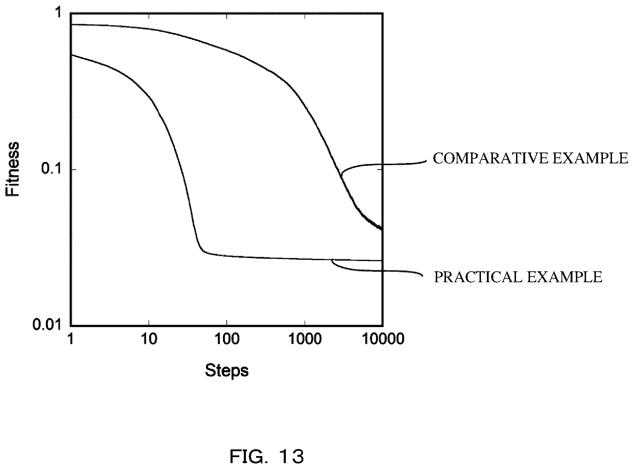

FIG.13 is a graph showing changes in Fitness of a practical example and a comparative example. For Fitness (degree of coincidence), the above-described formula (1) was used, and it was used as the convergence condition that Fitness is 0.05 or less. That is, in the practical example and the comparative example, the adaptation to the convergence condition was determined by directly using the mathematical expression for determining the degree of coincidence. The graph in FIG. 13 shows that it takes about 2.5 seconds per step. In the comparative example, it took 5000 steps as 12500 seconds to satisfy the convergence condition. On the other hand, in the practical example, only 50 steps as 125 seconds are required until the convergence condition is satisfied. The initial value of Fitness in the practical example was 0.633, and the final value was 0.026. On the other hand, the initial value of Fitness in the comparative example was 0.866, and the final value was 0.041. Thus, it was found that the method of the present invention converges about two orders of magnitude earlier than the conventional method in the case of a sample having a high degree of orientation.

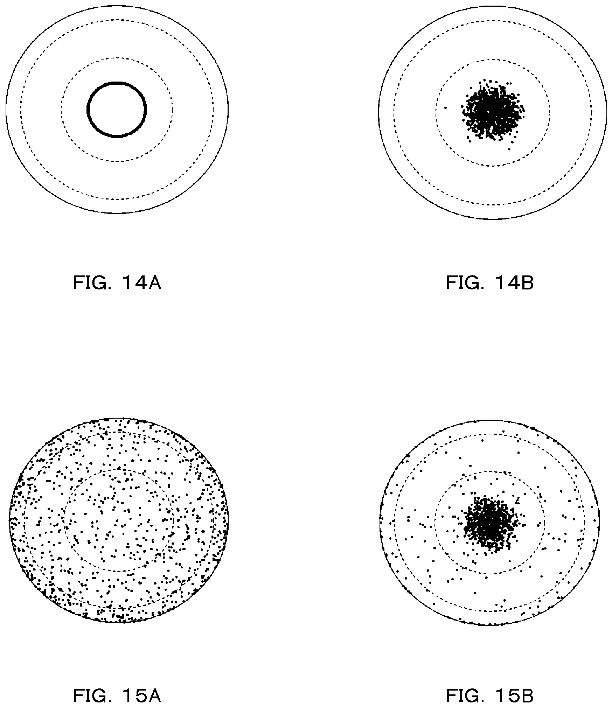

FIGS. 14A and 14B are a projection diagram showing the distribution of orientation directions in the initial-state and a projection diagram showing the distribution of orientation directions when the convergence condition is satisfied, respectively, in the practical example. FIGS. 15A and 15B are a projection diagram showing the distribution of orientation directions in the initial-state and a projection diagram showing the distribution of orientation directions when the convergence condition is satisfied, respectively, in the comparative example. In both cases, the orientation direction (the direction of the c-axis) is represented by a point on the spherical surface, and the orientation distribution is displayed by a projection diagram in the stretching direction. FIGS. 14A, 14B, 15A, and 15B show examples of the analysis of uniaxially oriented samples. The initial value for the orientation of the crystallites in FIG. 14A is $\theta_2=15°$ as described above, and $\theta_1$ and $\theta_3$ are not limited. Since there is no limit to $\theta_1$, a circular projection is obtained. Since the comparative example starts from a random state, it was found that the distribution of varied orientations other than the center occurs when the

# EP 4 597 088 A1

convergence condition is satisfied, resulting in an orientation with a local minimum.

From the above results, it was confirmed that the calculation apparatus, the method, and the program of the present invention can easily estimate the orientation direction from the two-dimensional diffraction image. In addition, it was confirmed that the total optimum solution was obtained by simulating with the RMC method using the estimated orientation direction as the initial state.

Needless to say, the present invention is not limited to the above-described embodiments. The scope of the present invention covers various modifications and equivalents included in the technical idea of the present invention. In addition, the names, structures, shapes, numbers, positions, sizes, and the like of the constituent elements shown in the drawings are for convenience of explanation and may be changed as appropriate.

The functionality of the elements disclosed herein may be implemented using circuitry or processing circuitry which includes general purpose processors, special purpose processors, integrated circuits, ASICs("Application Specific Integrated Circuits"), FPGAs("Field Programmable Gate Arrays"), conventional circuitry and/or combinations thereof which are programmed, using one or more programs stored in one or more memories, or otherwise configured to perform the disclosed functionality. Processors are considered processing circuitry or circuitry as they include transistors and other circuitry therein. The processor may be a programmed processor which executes a program stored in a memory. In the disclosure, the circuitry, units, or means are hardware that carry out or are programmed to perform the recited functionality. The hardware may be any hardware disclosed herein which is programmed or configured to carry out the recited functionality.

This application claims priority from Japanese Patent Application No. 2024-012292, filed on Jan. 30, 2024, the entire contents of Japanese Patent Application No. 2024-012292 are incorporated herein by reference.

Description of Symbols

100 calculation system
200 X-ray diffraction apparatus
210 X-ray generating section
220 incident side optical unit
230 goniometer
240 sample stage
250 emitting side optical unit
260 detector
300 control apparatus
310 control section
320 apparatus information storing section
330 measurement data storing section
340 display section
400 calculation apparatus
410 data acquiring section
415 crystalline component extracting section
420 initial structural model generating section
430 crystallite direction calculating section
440 storage section
450 pre-calculation structural model generating section
460 calculation executing section
510 input device
520 display device

**Claims**

1. A calculation apparatus for calculating a structural model of a polymer, comprising:

    a data acquiring section for acquiring a two-dimensional diffraction image and crystal structure data,
    an initial structural model generating section for generating an initial structural model including one crystallite, and
    a crystallite direction calculating section for calculating a direction of the crystallite,
    wherein the direction of the crystallite is calculated based on a two-dimensional diffraction image calculated from the initial structural model and the measured two-dimensional diffraction image.

2. The calculation apparatus according to claim 1,
   wherein the crystallite direction calculating section calculates a degree of coincidence between two-dimensional diffraction images calculated from the plurality of initial structural models corresponding to a plurality of directions of the one crystallite and the measured two-dimensional diffraction image and calculates the direction of the crystallite based on the calculated degree of coincidence.

3. The calculation apparatus according to claim 1 or 2, further comprising:

   a pre-calculation structural model generating section for generating a pre-calculation structural model in which the directions of the plurality of crystallites are the same direction for the structural model having the plurality of crystallites, and
   a calculation executing section for changing the structural model based on the degree of coincidence between the two-dimensional diffraction image calculated from the structural model and the measured two-dimensional diffraction image,
   wherein the calculation executing section calculates a post-calculation structural model in which the degree of coincidence satisfies a convergence condition using the pre-calculation structural model as a first structural model.

4. The calculation apparatus according to any one of claims 1 to 3, further comprising a crystal component extracting section for extracting a crystal component of the measured two-dimensional diffraction image,
   wherein the crystallite direction calculating section calculates the direction of the crystallite using the crystal component of the measured two-dimensional diffraction image.

5. The calculation apparatus according to any one of claims 1 to 4,
   wherein the direction of the crystallite is a three-dimensional vector.

6. The calculation apparatus according to claim 3,
   wherein the calculation executing section calculates the post-calculation structural model using the RMC method.

7. The calculation apparatus according to claim 3,

   wherein the pre-calculation structural model generating section selectively generates either the pre-calculation structural model or a second pre-calculation structural model in which directions of the plurality of crystallites are randomly arranged with respect to the structural model, and
   the calculation executing section calculates the post-calculation structural model using either the pre-calculation structural model or the second pre-calculation structural model as a first structural model.

8. A system comprising:

   an X-ray diffraction apparatus comprising an X-ray generator for generating X-rays, a detector for detecting X-rays and a sample stage for controlling the rotation of the sample, and
   the calculation apparatus according to any one of claims 1 to 7.

9. A method of calculating a structural model of a polymer, comprising the steps of:

   acquiring a two-dimensional diffraction image and crystal structure data,
   generating an initial structural model including one crystallite, and
   calculating a direction of the crystallite,
   wherein the direction of the crystallite is calculated based on a two-dimensional diffraction image calculated from the initial structural model and the measured two-dimensional diffraction image.

10. A program for calculating a structural model of a polymer, causing a computer to execute the following processing:

    acquiring a two-dimensional diffraction image and crystal structure data,
    generating an initial structural model including one crystallite, and
    calculating a direction of the crystallite,

wherein the direction of the crystallite is calculated based on a two-dimensional diffraction image calculated from the initial structural model and the measured two-dimensional diffraction image.

FIG. 1

FIG. 2

FIG. 3

FIG. 4

FIG. 5

CALCULATION APPARATUS  L  400

410  DATA ACQUIRING
SECTION

INITIAL STRUCTURAL
MODEL GENERATING
SECTION  420

430  CRYSTALLITE
DIRECTION
CALCULATING SECTION

STORAGE SECTION  440

450  PRE-CALCULATION
STRUCTURAL MODEL
GENERATING SECTION

CALCULATION
EXECUTING SECTION  460

INPUT DEVICE

DISPLAY DEVICE

510

520

FIG. 6

CALCULATION APPARATUS — 400

410 — DATA ACQUIRING SECTION

CRYSTALLINE COMPONENT EXTRACTING SECTION — 415

420 — INITIAL STRUCTURAL MODEL GENERATING SECTION

CRYSTALLITE DIRECTION CALCULATING SECTION — 430

440 — STORAGE SECTION

PRE-CALCULATION STRUCTURAL MODEL GENERATING SECTION — 450

460 — CALCULATION EXECUTING SECTION

INPUT DEVICE — 510

DISPLAY DEVICE — 520

L

FIG. 7

FIG. 8A

FIG. 8B

FIG. 8C

FIG. 8D

START

↓

ACQUIRING
TWO-DIMENSIONAL
DIFFRACTION IMAGE AND
CRYSTAL STRUCTURE DATA    S1

↓

GENERATING INITIAL
STRUCTURAL MODEL    S2

↓

CALCULATING
TWO-DIMENSIONAL
DIFFRACTION IMAGE FROM
INITIAL STRUCTURAL MODEL    S3

↓

CALCULATING DIRECTION OF
CRYSTALLITE    S4

↓

END

FIG. 9

FIG. 10

START

ACQUIRING TWO-DIMENSIONAL DIFFRACTION IMAGE AND CRYSTAL STRUCTURE DATA — U1

EXTRACTING CRYSTALLINE COMPONENT — U2

GENERATING INITIAL STRUCTURAL MODEL — U3

CALCULATING TWO-DIMENSIONAL DIFFRACTION IMAGE FROM INITIAL STRUCTURAL MODEL — U4

CALCULATING DIRECTION OF CRYSTALLITE — U5

GENERATING A PRE-CALCULATION STRUCTURAL MODEL — U6

CHANGING PRE-CALCULATION STRUCTURAL MODEL — U7

CALCULATING TWO-DIMENSIONAL DIFFRACTION IMAGE FROM PRE-CALCULATION STRUCTURAL MODEL — U8

CALCULATING DEGREE OF COINCIDENCE — U9

IS CONVERGENCE CONDITION SATISFIED? — U10

NO

YES

DECIDING POST-CALCULATION STRUCTURAL MODEL — U11

END

FIG. 11

FIG. 12A

FIG. 12B

FIG. 12C

FIG. 13

FIG. 14A

FIG. 14B

FIG. 15A

FIG. 15B

## EUROPEAN SEARCH REPORT

**Application Number**

EP 25 15 2406

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MURTHY N S ET AL: "Full-pattern parametrization of two-dimensional wide-angle diffraction data from oriented polymers", POLYMER, ELSEVIER, AMSTERDAM, NL, vol. 38, no. 9, 1 April 1997 (1997-04-01), pages 2277-2280, XP004058625, ISSN: 0032-3861, DOI: 10.1016/S0032-3861(96)00946-9 | 1,4,5, 8-10 | INV. G01N23/2055 G01N23/207 |
| Y | * the whole document * | 2,3,6,7 | |
| Y | ANDRZEJ BURIAN ET AL: "Structural studies of carbons by neutron and x-ray scattering", REPORTS ON PROGRESS IN PHYSICS, INSTITUTE OF PHYSICS PUBLISHING, BRISTOL, GB, vol. 82, no. 1, 21 November 2018 (2018-11-21), page 16501, XP020333003, ISSN: 0034-4885, DOI: 10.1088/1361-6633/AAE882 [retrieved on 2018-11-21] | 2,3,6,7 | |
| A | * page 12, paragraph 5.2 - page 17, paragraph 6; figures * | 1,4,5, 8-10 | |

**TECHNICAL FIELDS SEARCHED (IPC)**

G01N

The present search report has been drawn up for all claims

| Place of search | Date of completion of the search | Examiner |
|---|---|---|
| The Hague | 22 May 2025 | Savage, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

**Patent documents cited in the description**

- JP 2024012292 A

**Non-patent literature cited in the description**

- **R. L. MCGREEDY** ; **L. PUSZTAI**. Reverse Monte Carlo Simulation: A New Technique for the Determination of Disordered Structures. *Molecular Simulation*, 1988, vol. 1 (6), 359-367

- **DAISUKE TAWARA** ; **KOHJI TASHIRO**. Development of Polymer Three-Dimensional Higher Structural Analysis Based on Computer Simulation Technique of Measured Wide-Angle and Small-Angle X-Ray Scattering Data. *Proceedings of the Society of Polymer Science Japan*, 2018, vol. 67 (1)
- **Y.ZHANG**. Uncovering three-dimensional gradients in fibrillar orientation in an impact-resistant biological armour. *Scientific Reports*, 2016, vol. 6, 26249